# EUROPEAN PATENT APPLICATION

(11) **EP 1 669 448 A1**
(43) Date of publication of application: **14.06.2006**
(21) Application number: 04788077.8
(22) Date of filing: 24.09.2004
(51) Int. Cl.: C12N 15/09, C07K 14/445, C07K 16/20, C12Q 1/68, G01N 33/50, G01N 33/15, A61K 39/002, A61K 39/395

(54) **PROCESS FOR PRODUCING ANTIGENIC SUBSTANCE**

(30) Priority: 25.09.2003 JP 2003333659
(71) Applicant: CellFree Sciences Co., Ltd., Yokohama-shi, Kanagawa 230-0046 (JP)
(72) Inventor: ENDO, Yaeta, Matsuyama-shi, Ehime 7918016 (JP); TSUBOI, Takafumi, Onsen-gun, Ehime 7910202 (JP); TORII, Motomi, Iyo-gun, Ehime 7912114 (JP); SAWASAKI, Tatsuya, Matsuyama-shi, Ehime 7900811 (JP)
(74) Representative: Forstmeyer, Dietmar
(86) International application number: PCT/JP2004/013918
(87) International publication number: WO 2005/030954

(57) **Abstract**

An object of the present invention is to provide a means for producing an antigenic component with the retained native antigenicity using a cell-free protein synthesis. In particular, it is an object to provide a means for producing an antigenic component without depending on codon usage, like expressing an antigenic component from a gene containing a large amount of AT.

The present inventors have made a strenuous study to solve the matters described above and successfully completed the present invention by preparing an antigenic component with the retained antigenicity, in particular a malaria antigen useful for manufacturing a malaria vaccine, through a system with the use of a wheat embryo among cell-free protein synthesis means.

SEQUENCE LISTING

<110>  Cell Free Science Co., Ltd.

<120>  A method of antigen production

<130>  GP04-1016

<150>  JP 2003-0925
<151>  2003-333659

<160>  16

<170>  PatentIn version 3.1

<210>  1
<211>  561
<212>  DNA
<213>  Plasmodium falciparum

<400>  1
atgagcataa aatataataa tgcgaaagtt accgtggata ctgtatgcaa aagaggattt      60

ttaattcaga tgagtggtca tttggaatgt aaatgtgaaa atgatttggt gttagtaaat     120

gaagaaacat gtgaagaaaa agttctgaaa tgtgacgaaa agactgtaaa taaaccatgt     180

ggagattttt ccaaatgtat taaaatagat ggaaatcccg tttcatacgc ttgtaaatgt     240

aatcttggat atgatatggt aaataatgtt tgtataccaa atgaatgtaa gaatgtaact     300

tgtggtaacg gtaaatgtat attagataca agcaatcctg ttaaaactgg agtttgctca     360

tgtaatatag gcaaagttcc caatgtacaa gatcaaaata aatgttcaaa agatggagaa     420

accaaatgct cattaaaatg cttaaaagaa aatgaaacct gtaaagctgt tgatggaatt     480

tataaatgtg attgtaaaga tggatttata atagataatg aaagctctat atgtactgct     540

caccaccacc accaccacta g                                               561

<210>  2
<211>  186
<212>  PRT
<213>  Plasmodium falciparum

<400>  2

Met Ser Ile Lys Tyr Asn Asn Ala Lys Val Thr Val Asp Thr Val Cys
1               5                   10                  15

Lys Arg Gly Phe Leu Ile Gln Met Ser Gly His Leu Glu Cys Lys Cys
            20                  25                  30

Glu Asn Asp Leu Val Leu Val Asn Glu Glu Thr Cys Glu Glu Lys Val
        35                  40                  45

Leu Lys Cys Asp Glu Lys Thr Val Asn Lys Pro Cys Gly Asp Phe Ser
    50                  55                  60

Lys Cys Ile Lys Ile Asp Gly Asn Pro Val Ser Tyr Ala Cys Lys Cys
65                  70                  75                  80

Asn Leu Gly Tyr Asp Met Val Asn Asn Val Cys Ile Pro Asn Glu Cys
                85                  90                  95

Lys Asn Val Thr Cys Gly Asn Gly Lys Cys Ile Leu Asp Thr Ser Asn
            100                 105                 110

Pro Val Lys Thr Gly Val Cys Ser Cys Asn Ile Gly Lys Val Pro Asn
        115                 120                 125

Val Gln Asp Gln Asn Lys Cys Ser Lys Asp Gly Glu Thr Lys Cys Ser
    130                 135                 140

Leu Lys Cys Leu Lys Glu Asn Glu Thr Cys Lys Ala Val Asp Gly Ile
145                 150                 155                 160

Tyr Lys Cys Asp Cys Lys Asp Gly Phe Ile Ile Asp Asn Glu Ser Ser
                165                 170                 175

Ile Cys Thr Ala His His His His His His
            180                 185

<210>  3
<211>  540
<212>  DNA
<213>  Plasmodium falciparum

<400>  3
atggtaacag tcgacaccgt ctgtaagaga ggtttcttga ttcaaatgtc cggtcacttg      60

gaatgtaagt gtgaaaacga cttggtcttg gttaacgaag aaacttgtga agaaaaggtc     120

ttgaagtgtg acgaaaagac tgtcaacaag ccatgtggtg acttctctaa gtgtatcaag     180

atcgatggta acccagtctc ttacgcctgt aagtgtaact tgggttacga tatggtcaac     240

aacgtctgta ttccaaacga atgtaagcaa gttacctgtg gtaacggtaa gtgtatcttg     300

gatacttcca acccagttaa gaccggtgtt tgttcttgta acattggtaa ggtcccaaac     360

gttcaagacc aaaacaagtg ttctagagac ggtgaaacta agtgttcctt gaagtgtttg     420

aaggaacaag aaacctgtaa ggctgttgac ggtatttaca agtgtgactg taaggatggt     480

ttcatcattg accaagaatc ttccatttgt accgatccac accaccacca ccaccactag     540

<210>  4
<211>  179
<212>  PRT
<213>  Plasmodium falciparum

<400>  4

Met Val Thr Val Asp Thr Val Cys Lys Arg Gly Phe Leu Ile Gln Met
1               5                   10                  15

Ser Gly His Leu Glu Cys Lys Cys Glu Asn Asp Leu Val Leu Val Asn
            20                  25                  30

Glu Glu Thr Cys Glu Glu Lys Val Leu Lys Cys Asp Glu Lys Thr Val
        35                  40                  45

Asn Lys Pro Cys Gly Asp Phe Ser Lys Cys Ile Lys Ile Asp Gly Asn
    50                  55                  60

Pro Val Ser Tyr Ala Cys Lys Cys Asn Leu Gly Tyr Asp Met Val Asn
65                  70                  75                  80

Asn Val Cys Ile Pro Asn Glu Cys Lys Gln Val Thr Cys Gly Asn Gly

                85                  90                  95

Lys Cys Ile Leu Asp Thr Ser Asn Pro Val Lys Thr Gly Val Cys Ser
            100                 105                 110

Cys Asn Ile Gly Lys Val Pro Asn Val Gln Asp Gln Asn Lys Cys Ser
        115                 120                 125

Arg Asp Gly Glu Thr Lys Cys Ser Leu Lys Cys Leu Lys Glu Gln Glu
    130                 135                 140

Thr Cys Lys Ala Val Asp Gly Ile Tyr Lys Cys Asp Cys Lys Asp Gly
145                 150                 155                 160

Phe Ile Ile Asp Gln Glu Ser Ser Ile Cys Thr Asp Pro His His His
                165                 170                 175

His His His

<210> 5
<211> 555
<212> DNA
<213> Plasmodium vivax

<400> 5
atggctagcg ccgtcacggt agacaccata tgcaaaaatg gacagctggt tcaaatgagt 60

aaccacttta agtgtatgtg taacgaaggg ctggtgcacc tttccgaaaa tacatgtgaa 120

gaaaaaaatg aatgcaagaa agaaacccta ggcaaagcat gcggggaatt tggccagtgt 180

atagaaaacc cagacccagc acaggtaaac atgtacaaat gtggttgcat tgagggctac 240

actttgaagg aagacacttg tgtgcttgat gtatgtcaat acaaaaattg tggagaaagt 300

ggcgaatgca ttgttgagta cctctcggaa atccaaagtg caggttgctc atgtgctatt 360

ggcaaagtcc ccaatccaga agatgagaaa aaatgtacca aaacgggaga aactgcttgt 420

caattgaaat gtaacacaga taatgaagtc tgcaaaaatg ttgaaggagt ttacaagtgc 480

cagtgtatgg aaggctttac gttcgacaaa gagaaaaatg tatgccttgg gccccaccac 540

caccaccacc actga 555

<210> 6
<211> 184
<212> PRT
<213> Plasmodium vivax

<400> 6

Met Ala Ser Ala Val Thr Val Asp Thr Ile Cys Lys Asn Gly Gln Leu
1               5                   10                  15

Val Gln Met Ser Asn His Phe Lys Cys Met Cys Asn Glu Gly Leu Val
            20                  25                  30

His Leu Ser Glu Asn Thr Cys Glu Glu Lys Asn Glu Cys Lys Lys Glu
        35                  40                  45

Thr Leu Gly Lys Ala Cys Gly Glu Phe Gly Gln Cys Ile Glu Asn Pro
    50                  55                  60

Asp Pro Ala Gln Val Asn Met Tyr Lys Cys Gly Cys Ile Glu Gly Tyr
65                  70                  75                  80

Thr Leu Lys Glu Asp Thr Cys Val Leu Asp Val Cys Gln Tyr Lys Asn
                85                  90                  95

Cys Gly Glu Ser Gly Glu Cys Ile Val Glu Tyr Leu Ser Glu Ile Gln
            100                 105                 110

Ser Ala Gly Cys Ser Cys Ala Ile Gly Lys Val Pro Asn Pro Glu Asp
        115                 120                 125

Glu Lys Lys Cys Thr Lys Thr Gly Glu Thr Ala Cys Gln Leu Lys Cys
    130                 135                 140

Asn Thr Asp Asn Glu Val Cys Lys Asn Val Glu Gly Val Tyr Lys Cys
145                 150                 155                 160

Gln Cys Met Glu Gly Phe Thr Phe Asp Lys Glu Lys Asn Val Cys Leu
                165                 170                 175

Gly Pro His His His His His His
            180

<210>  7
<211>  612
<212>  DNA
<213>  Plasmodium vivax

<400>  7
atggctagca aggtcaccgc ggagacccaa tgcaaaaatg gctatgtagt ccaaatgagc      60

aatcattttg aatgcaaatg caacgacggg tttgttctgg caaatgaaaa cacttgcgag     120

gaaaaacgcg attgcacaaa tccacaaaat gtaaataaaa actgtggaga ctacgctgtg     180

tgtgcaaaca ccagaatgaa taatgaggaa agagcattac gatgcggctg catattaggg     240

tacaccgtaa tgaatgaggt gtgtactcca tataaatgta acggcgttct gtgtggaaag     300

ggaaagtgca tcttggatcc cgctaatgtg caaagcacca tgtgctcttg taatatagga     360

agcacattgg atgaatctaa aaaatgtgga aagccaggaa aaactgaatg cacgttgaag     420

tgtaaggcaa acgaagaatg taaagagact cagaattatt acaagtgcgt tgcgaaggga     480

agcggcggag aaggcagcgg tggagaaggc agcggtggag aaggcagcgg cggagagggc     540

agcggcggag agggcagcgg tggagacaca ggagcagctt acagtgggcc ccaccaccac     600

caccaccact ga                                                         612

<210>  8
<211>  203
<212>  PRT
<213>  Plasmodium vivax

<400>  8

Met Ala Ser Lys Val Thr Ala Glu Thr Gln Cys Lys Asn Gly Tyr Val
1               5                   10                  15

Val Gln Met Ser Asn His Phe Glu Cys Lys Cys Asn Asp Gly Phe Val

20                  25                  30

Leu Ala Asn Glu Asn Thr Cys Glu Glu Lys Arg Asp Cys Thr Asn Pro
        35                  40                  45

Gln Asn Val Asn Lys Asn Cys Gly Asp Tyr Ala Val Cys Ala Asn Thr
    50                  55                  60

Arg Met Asn Asn Glu Glu Arg Ala Leu Arg Cys Gly Cys Ile Leu Gly
65                  70                  75                  80

Tyr Thr Val Met Asn Glu Val Cys Thr Pro Tyr Lys Cys Asn Gly Val
                85                  90                  95

Leu Cys Gly Lys Gly Lys Cys Ile Leu Asp Pro Ala Asn Val Gln Ser
            100                 105                 110

Thr Met Cys Ser Cys Asn Ile Gly Ser Thr Leu Asp Glu Ser Lys Lys
        115                 120                 125

Cys Gly Lys Pro Gly Lys Thr Glu Cys Thr Leu Lys Cys Lys Ala Asn
    130                 135                 140

Glu Glu Cys Lys Glu Thr Gln Asn Tyr Tyr Lys Cys Val Ala Lys Gly
145                 150                 155                 160

Ser Gly Gly Glu Gly Ser Gly Gly Glu Gly Ser Gly Gly Glu Gly Ser
                165                 170                 175

Gly Gly Glu Gly Ser Gly Gly Glu Gly Ser Gly Gly Asp Thr Gly Ala
            180                 185                 190

Ala Tyr Ser Gly Pro His His His His His His
        195                 200

<210>  9
<211>  24
<212>  DNA
<213>  Artificial sequence

<220>
<223>  Primer for cloning Pfs25-3D7 antigen gene

<400>  9
atgagcataa aatataataa tgcg                                              24

<210>  10
<211>  42
<212>  DNA
<213>  Artificial sequence

<220>
<223>  Primer for cloning Pfs25-3D7 antigen gene

<400>  10
ctagtggtgg tggtggtggt gagcagtaca tatagagctt tc                          42

<210>  11
<211>  29
<212>  DNA
<213>  Artificial sequence

<220>
<223>  Primer for cloning Pfs25-TBV antigen gene

<400>  11
atggtaacag tcgacaccgt ctgtaagag                                          29

<210>  12
<211>  20
<212>  DNA
<213>  Artificial sequence

<220>
<223>  Primer for cloning Pfs25-TBV antigen gene

<400>  12
gaacagtcat gtctaaggcg                                                    20

<210>  13
<211>  26
<212>  DNA
<213>  Artificial sequence

<220>
<223>  Primer for cloning Pvs25 antigen gene

<400>  13
atggctagcg ccgtcacggt agacac                                             26

<210>  14
<211>  45
<212>  DNA
<213>  Artificial sequence

<220>
<223>  Primer for cloning Pvs25 antigen gene

<400>  14
tcagtggtgg tggtggtggt ggggcccaag gcatacattt ttctc                        45

<210>  15
<211>  21
<212>  DNA
<213>  Artificial sequence

<220>
<223>  Primer for cloning Pvs28 antigen gene

<400>  15
atggctagca aggtcaccgc g                                                  21

<210>  16
<211>  41
<212>  DNA
<213>  Artificial sequence

<220>
<223>  Primer for cloning Pvs28 antigen gene

<400>  16
tcagtggtgg tggtggtggt ggggcccact gtaagctgct c                            41

## Description

### Technical Field

The present invention relates to a method for producing an antigenic component in a cell-free protein synthesis with the use of a wheat embryo. More specifically, the present invention relates to a method for producing an antigenic component encoded by a gene having an AT content of 50% or more, in particular a method for producing an antigenic component derived from a plasmodium, and an antigenic component obtained by the producing method, a vaccine comprising the antigenic component, an antibody prepared by the antigenic component.

### Background Art

As a method for practicing intracellular protein synthesis ex vivo such as in a test tube, for example, the method of extracting ribosome and other components necessary for protein synthesis from an organism (herein, the extract may be referred to as "a wheat embryo extract for cell-free protein synthesis") and using them to carry out synthesis in a cell-free protein in vitro has been extensively studied (patent document Nos.1, No.2, No.3, No.4, and No.5).

The cell-free protein synthesis system is a useful method, which has comparably excellent performances to a living cell system in accuracy and rate of translation reaction and can provide an aimed protein without carrying out a complicated purification step. Therefore, the application of the synthesis system for better industrial uses needs providing a wheat embryo extract-containing solution for synthesis and a ready-made type wheat embryo extract-containing solution steadily with the retained high quality, in addition to increasing the synthesis efficiency.

Meanwhile, malaria has become a threat to humankind because the drug tolerant plasmodia appears to increase the number of the patients around the world. But, there has been put no vaccine into practical use yet. In the year of 2002, the decoding of the genome of Plasmodium falciparum was completed to identify more than 5000 genes, environmentally allowing development of the genome-extensive research. However, the research on malaria so far meets a critical obstacle that it must cope with many recombinant proteins which are difficult to express in the existing system. Consequently, in order to develop a vaccine for the disease, researchers including the present inventors can not help limiting to the proteins of protozoa that can be expressed in the existing systems.

Until now, a group in the National Institutes of Health of the United State has challenged various expression systems in association with Pfs25 and obtained a large amount of recombinant proteins whose conformations are relatively analogous to the proteins of protozoa, through an expression system using artificially synthesized genes and yeasts. In 1997, the phase I clinical trial of a vaccine using Pfs25, which was synthesized by this method, for preventing the propagation of falciparum malaria was carried out in the United States. As a result, although the antibodies in the blood serums of volunteers were shown to have inhibitory activity to the propagation, the activity was at most as low as 50%, leaving problems to be solved for practical applications (non-patent document No.1).
Meanwhile, in the West which has primarily aimed a countermeasure at falciparummalaria in the African region, little attention has been paid to and no research has been conducted for the development of vaccines for preventing the propagation of vivax malaria which occurs as epidemic as falciparum malaria in the other tropical regions than Africa. Therefore, we addressed ourselves to the development of a vaccine to prevent vivax malaria propagation. After cloning the genes, Pvs25 and Pvs28, (non-patent document No.2, patent document No.6), the recombinant proteins were expressed in a yeast, and then animals were immunized with them. As a result, these recombinant proteins were found to induce effective antibodies for preventing propagation. [(non-patent document No.3), (non-patent document No.4)].
Because falciparum malaria exists in the most of regions where vivax malaria is epidemic, it is believed that different vaccines against the both protozoa are important to develop simultaneously for their practical applications. Further, plasmodia have a mechanism for exempting themselves from the immunization of hosts, such as antigenic variation and polymorphism, and hence it is said that the practical application needs a plurality of candidate antigens to prepare for vaccines. However, today, there are studied only three kinds of proteins which are considered as candidates for vaccines to prevent propagation of Plasmodium falciparum and Plasmodium vivax (non-patent document No.5).

Despite of such situation in malaria vaccine as described above, until now it has been believed that a promising antigenic component is difficult to produce by a cell-free synthesis means because of problems from the conformation of a produced protein and from a added sugar chain.
Further, it is known that an antibody produced through a cell-free protein synthesis system derived from E. Coli cannot keep a sufficient conformation necessary to recognize the antigen, resulting in a low Kd value (non-patent document No. 6, 7).

[patent document No.1] Japanese patent Application Laid-open No. Hei 6-98790
[patent document No.2] Japanese patent Application Laid-open No. Hei 6-225783
[patent document No.3] Japanese patent Application Laid-open No. Hei 7-194
[patent document No.4] Japanese patent Application Laid-open No. Hei 9-291
[patent document No.5] Japanese patent Application Laid-open No. Hei 7-147992
[patent document No.6] International Patent Application PCT/US98/25742
[non-patent document No.1] D.C. Kaslow, Transmission-blocking vaccines.
P. Perlmann, M. Troye-Blomberg (eds): Malaria immunology. Chem. Immunol. Basel, vol. 80, 287-307, 2002.
[non-patent document No.2] T. Tsuboi, D.C. Kaslow, M.M.G. Gozar, M. Tachibana, Y-M. Cao, M. Torii, Sequence polymorphism in two novel Plasmodium vivax ookinete surface proteins,Pvs25 and Pvs28, that are malaria transmission-blocking vaccine candidates. Mol. Med. 4, 772-782, 1998
[non-patent document No.3] H. Hisaeda, A.W. Stowers, T. Tsuboi, W.E. Collins, J. Sattabongkot, N.Suwanabun, M. Torii, D.C. Kaslow Antibodies to malaria vaccine candidates Pvs25 and Pvs28 completely block theability of Plasmodium vivax to infect mosquitoes.
   Infect. Immun. 68, 6618-6623, 2000.
[non-patent document No.4] Arakawa, T. Tsuboi, A. Kishimoto, J. Sattabongkot, N. Suwanabun, T.Rungruang, Y. Matsumoto, N. Tsuji,
   H. Hisaeda, A. Stowers, I. Shimabukuro, Y. Sato, M. Torii Serum antibodies induced by intranasal immunization of mice with Plasmodiumvivax Pvs25 co-administered with cholera toxin completely block parasitetransmission to mosquitoes. Vaccine 21: 3143-3148, 2003.
[non-patent document No.5] T. Tsuboi, M. Tachibana, O. Kaneko, M.Torii
   Transmission-blocking vaccine of vivax malaria.
   Parasitol. Int. 52: 1-11, 2003.
[non-patent document No. 6] ALEXANDER ZDANOV., et al., Proc. Natl. Acad. Sci. USA., Vol 91,pp.6423-6427 (1994),
[non-patent document No.7] C.Roger Mackenzie. , et al., THE JOURNAL OF BIOLOGICAL CHEMISTRY., Vo1271, pp.1527-1533 (1998)

### Disclosure of the Invention

An object of the present invention is to provide a means for producing an antigenic component with the retained native antigenicity using a cell-free protein synthesis means. In particular, it is an object to provide a means for producing an antigenic component without depending on codon usage, like expressing an antigenic component from a gene containing a large amount of AT.

The present inventors have made a strenuous study to solve the matters described above and successfully completed the present invention by preparing an antigenic component with the retained antigenicity, in particular a malaria antigen useful for manufacturing a malaria vaccine, through a system with the use of a wheat embryo among cell-free protein synthesis means.

Therefore, the present invention is composed of:
1. A method for producing an antigenic component using a cell-free protein synthesis means with the use of a wheat embryo extract.
2. A method for producing an antigenic component which is encoded by a gene having an AT content of 50% or more by a base sequence of its protein-coding region, using a cell-free protein synthesis means with the use of a wheat embryo extract.
3. A method for producing an antigenic component derived from a plasmodium using a cell-free protein synthesis means with the use of a wheat embryo extract.
4. The method for producing according to any one of the preceding 1 to 3, wherein the cell-free protein synthesis means uses a wheat embryo extract which is substantially freed from an endosperm and a low molecular synthesis inhibitor.
5. An antigenic component obtained by the method for producing according to the preceding 4.
6. The antigenic component according to the preceding 5, wherein the antigenic component has an amino acid sequence which has the identity or a homology of 70% or more to the amino acid sequence of SEQ. ID. No: 2, 4, 6 or 8.
7. The antigenic component according to the preceding 6, wherein the antigenic component retains the antigenicity.
8. A method for manufacturing a vaccine using the antigenic component according to any one of the preceding 5 to 7.
9. A vaccine obtained by the method for manufacturing according to the preceding 8, wherein the vaccine comprises the antigenic component.
10. An antibody prepared by the vaccine according to the preceding 9.
11. An antibody which reacts immunologically with the antigenic component according to any one of the preceding 5 to 7.
12. A diagnostic agent comprising the antibody according to the preceding 10 or 11.
13. A diagnostic kit comprising the diagnostic agent according to the preceding 12.
14. A method for screening an antigenic component using a cell-free protein synthesis means with the use of a wheat embryo extract, comprising:
   1) a step for preparing a gene comprising a region selected within a desired candidate gene,
   2) a step for synthesizing a mRNA from the gene prepared in step 1),
   3) a step for synthesizing a polypeptide by a cell-free protein synthesis means with the use of a wheat embryo extract through translation using the mRNA synthesized in step 2) as a translation template, or synthesizing a polypeptide by a cell-free protein synthesis system with an integrated transcription/translation manner using the gene prepared in step 1) as a template,
   4) a step for immunizing a mammal with the polypeptide synthesized in step 3) to obtain the antiserum,
   5) a step for reacting the antiserum described above with a desired target microbe or a protein derived from the microbe to analyze the reactivity, and
   6) a step for identifying the region selected within the candidate gene from which the antiserum has been derived as a candidate antigenic component if the antiserum is confirmed to react.

A method for producing an antigenic component using a cell-free protein synthesis means with the use of a wheat embryo extract of the present invention makes it possible to provide an antigenic component with the retained native antigenicity through a cell-free protein synthesis system for the first time. Further, as revealed in the present invention, any AT content that a gene encoding a protein to synthesize has will not affect the protein synthesis in a cell-free protein synthesis system with the use of a wheat embryo. That is to say, it was revealed that even a gene having a high AT content allows protein synthesis in the synthesis system with no altered codon. As a result, the present invention provides a cell-free synthesis means for a wide range of antigenic components without limitation by codon usage and permits a method for producing antigenic components such as vaccines through a cell-free synthesis system.

### Description of the Preferred Embodiment

(1) Preparation of Wheat Embryo Extract-containing solution for Cell-free Protein Synthesis
In the present invention, a wheat embryo extract is used in a cell-free protein synthesis system. Herein, the cell-free protein synthesis system means an in vitro synthesis method carried out by extracting components including, for example, ribosome, which is an intracellularly-equipped protein translation apparatus, from wheat embryo, and adding the transcription template or translation template, nucleic acids as substrate, amino acids, energy source, various ions, buffer, and other useful factors to this extract solution. The system includes a system wherein RNA is used as a template to react (hereinafter sometimes referred to as "cell-free translation system") and a system wherein DNA is used and an enzyme necessary for transcription such as RNA polymerase is further supplied to react (hereinafter sometimes referred to as "cell-free transcription/translation system"). The cell-free protein synthesis system in the present invention includes both of a cell-free translation system and a cell-free transcription/translated system described above.

The wheat embryo extract-containing solution used in the present invention is commercially available as PROTEIOS^{™} (from TOYOBO). As an isolation method of a wheat embryo in a preparation method of a wheat embryo extract solution, the method described in, for example, Johnston, F. B. et al., Nature, 179, 160-161 (1957) may be used, and as an extraction method for a wheat embryo extract-containing solution from the isolated wheat embryo, for example, the method described in Erickson, A. H. et al., (1996) Meth. In Enzymol. , 96, 38-50 may be used. Besides them, the methods described in international patent application PCT/JP03/00995 may be mentioned.

The wheat embryo extract suitable for use in the present invention will be purified to be almost free from an endosperm containing substances (the substances such as tritin, thionine, and ribonuclease which act on mRNAs, tRNAs, protein translation factors, ribosome and others to inhibit those functions) which inhibit protein synthesis function that cells as materials have or keep in themselves. Herein, the wheat embryo extract purified to be almost free from an endosperm means a wheat embryo extract freed from an endosperm portion to such an extent that substantially no ribosome may be deadenylated. Such an extent that substantially no ribosome may be deadenylated means that a rate of less than 7% and preferably 1% or below of ribosome is deadenylated.

The wheat embryo extract described above may comprise a protein derived from a wheat embryo extract-containing solution (and independently supplied if necessary). In view of the preservation stability in a freeze-dried state, its usability and the like, the composition before freeze-dried comprises the protein preferably at a content of 1 to 10 weight%, and more preferably 2.5 to 5 weight% of the total composition, while the composition after freeze-dried comprises preferably the protein at a content of 10 to 90 weight%, and more preferably 25 to 70 weight% of the total lyophilized composition. But the content is not particularly limited. Now, the protein content herein can be calculated by determining its absorbance (260, 280 and 320 nm).

(2) Reduction of Deliquescent Substances in Wheat Embryo Extract-Containing Solution
The wheat embryo extract-containing solution described above contains extraction solvents or deliquescent substances such as potassium acetate and magnesium acetate originating from buffers used in gelfiltration after extraction. Thus, the wheat embryo extract-containing solution is used to prepare a translation reaction solution, which is then directly lyophilized to make a dry formulation with a trouble such as deliquescence, resulting in deterioration in quality of the formulation. Deterioration in quality means that the formulation may not be completely dissolved in added water, thereby to decrease synthesis activities in protein synthesis reaction. Therefore, the deliquescent substances in the wheat embryo extract-containing solution may be decreased in concentration to give no influence on the quality of the lyophilized formulation. The specific methods for reducing deliquescent substances include, for example, a gelfiltration method using a gel support equilibrated beforehand with a deliquescent substance-reduced or free solution, and a dialysis method. The deliquescent substances are reduced by using these methods to provide a solution for translation reaction which has a final deliquescent substance concentration of 60 mM or below. Specifically, the solution finally prepared for translation reaction should contain potassium acetate at a decreased concentration of 60 mM or below and preferably 50 mM or below. Furthermore, the lyophilized formulation has preferably a deliquescent substance content of 0.01 parts by weight or below to 1 part by weight of the protein contained in the formulation, and particularly preferably 0.005 parts by weight or below to keep the preservation stability in the freeze-dried state.

(3) Elimination of Contaminant Microbes
As the wheat embryo extract-containing solution can be contaminated with spores of microbes, in particular a filamentous bacterium (mold) and the like, these microbes are preferred to be eliminated. Microbial proliferation may be seen in particular in a long-term (a day or higher) cell-free protein synthesis reaction, and hence is important to inhibit. The means of eliminating microbes preferably includes, but is not limited to, a filter for filter sterilization to use. The filter may not be limited in pore size to a particular one in so far as it can eliminate contamination-suspected microbes, but has usually a pore size of 0.1 to 1 µm, and preferably 0.2 to 0.5 µm.

(4) Method to Remove Low Molecular Synthesis Inhibitors from Wheat Embryo Extract-containing Solution
In addition to the foregoing operations, a step for removing low molecular synthesis inhibitors can be added anywhere in the preparation of the wheat embryo extract-containing solution to make the solution suitable for cell-free protein synthesis of an antigenic component with more preferable effects.
A wheat embryo extract-containing solution prepared by substantially removing an endosperm component contains low molecular synthesis inhibitors having a protein synthesis inhibitory activity (this may be referred to as "low molecular synthesis inhibitor"). Thus, the removal of them may provide a wheat embryo extract-containing solution having a high protein synthesis activity. Specifically, the removal is conducted by fractionally removing low molecular synthesis inhibitors from the components of a wheat embryo extract-containing solution through the differences in their molecular weights. The low molecular synthesis inhibitor can be fractionally removed to have a smaller molecular weight than the least factor among those factors necessary for protein synthesis that are contained in the wheat embryo extract-containing solution. Specifically, the inhibitor may be fractionally removed to have a molecular weight of 50,000 to 14,000 or less, or preferably of less than 14, 000. As the method for removing low molecular synthesis inhibitors from a wheat embryo extract-containing solution, a method already known per se, for example, dialysis using a dialysis membrane, gelfiltration, or ultrafiltration can be used. Among them, a dialysis is preferred in view of, for example, the easiness of supplying an internal solution with materials.

As a dialysis membrane for use in the removing operation of low molecular synthesis inhibitors through dialysis, the one which can remove a molecule having a molecular weight of 50,000 to 12,000 may be mentioned, specifically, a recyclable cellulose membrane (from Viskase Sales, Chicago) which can remove a molecule having a molecular weight of 12, 000 to 14, 000, Spectra/Pore 6 (from SPECTRUM LABOTRATORIES INC., CA, USA) which can remove a molecule having a molecular weight of 50,000, and the like may preferably be used. A suitable amount of the wheat embryo extract-containing solution will be put toward the one-side of such dialysis membrane, and then dialysis is conducted by a conventional method. The dialysis is preferred to be conducted for 30 minutes to 24 hours.

While removing low molecular synthesis inhibitors, if an insoluble substance is produced in a wheat embryo extract-containing solution, inhibiting this production (hereinafter, this may be referred to as "the stabilization of a wheat embryo extract-containing solution") allows the wheat embryo extract-containing solution finally prepared or a solution for translation reaction to have a higher protein synthesis activity. As a specific method of stabilizing a wheat embryo extract-containing solution or a solution for translation reaction, there is mentioned a method wherein low molecular synthesis inhibitors described above are removed from a wheat embryo extract-containing solution or a solution for translation reaction with at least a high energy phosphate compound such as ATP or GTP (hereinafter, they may be referred to as "stabilization component") contained. As the high energy phosphate compound, ATP may preferably be used. Further, the removal may be preferably carried out from the solution with ATP and GTP, and more preferably ATP, GTP, and 20 kinds of amino acids contained.

The solution may be supplied with these stabilization components, incubated, and then subjected to the process for removing low molecular synthesis inhibitors. Alternatively, the stabilization component may be added also to an external solution for dialysis, and then the solution is subjected to dialysis to remove low molecular synthesis inhibitor. Advantageously, the stabilization component in the external solution for dialysis, even if decomposed during dialysis, can be constantly supplemented with the fresh stabilization component. This approach can be applied to gelfiltration and ultrafiltration used to give the same effect. The supports they use are equilibrated with a filtration buffer with a stabilization component contained, supplied with a wheat embryo extract-containing solution or a solution for translation reaction with a stabilization component contained, and the supplied with the above-described buffer to filtrate.

The amount of a stabilization component to add and the time of stabilization to treat may be selected as appropriate depending on the kind of a wheat embryo extract-containing solution and the method of preparation. As the selection method, there may be mentioned a method wherein a wheat embryo extract-containing solution is supplied with various stabilization components in amount and kind on a trial basis, and subjected to the step for removing low molecular synthesis inhibitors after an appropriate hour to give a treated wheat embryo extract-containing solution, which is then centrifuged to separate into the soluble compartment and the insoluble component, thereby to select a case resulting in a less amount of the insoluble component. Alternatively, a method is preferred wherein the treated wheat embryo extract-containing solution is used to carry out cell-free protein synthesis, thereby to select a case resulting in a high protein synthesis activity. Further, a method is mentioned wherein stabilization component is added in an external solution for dialysis, and then wheat embryo extract-containing solution is subjected to dialysis for an appropriate time, thereby to select based of the amount of the insoluble component in the solution thus obtained or the protein synthesis activity of the solution thus obtained.

As one example of the stabilizing condition of a wheat embryo extract-containing solution thus selected, specifically, if dialysis is carried out for the step of removing low molecular synthesis inhibitors, there is mentioned a method wherein the wheat embryo extract-containing solution and the external solution for dialysis are supplied with 100 µM to 0.5 mM of ATP, 25 µM to 1 mM of GTP and 25 µM to 5 mM of each 20 kinds of amino acids and then subjected to dialysis for 30 minutes to an hour or more. The temperature for dialysis may be any temperature so far as it does not deteriorate the protein synthesis activity of the wheat embryo extract-containing solution and allows dialysis. Specifically, the lowest temperature is a temperature at which the solution will not freeze, usually -10 °C, and preferably -5°C. The highest temperature is a limit temperature which gives no bad influence on the solution used in dialysis, 40 °C, and preferably 38 °C.

In addition, if low molecular synthesis inhibitors are removed after a wheat embryo extract-containing solution is prepared, the solution needs no further the above stabilization component to add.

(5) Method for Decreasing Concentration of Reducing Agent in Wheat Embryo Extract-containing Solution
The wheat embryo extract-containing solution, which contains a reducing agent at a decreased concentration, is used to execute cell-free protein synthesis, allowing production of a target antigenic component which has an intramolecularly formed disulfide bond. For a method for decreasing a reducing agent in a wheat embryo extract-containing solution, there is used a method wherein a step of decreasing a reducing agent is employed anywhere in the steps for the preparation of the wheat embryo extract-containing solution. The reducing agent should be decreased to have so a concentration in the finally prepared wheat embryo extract-containing solution that the solution may be used to execute translation reaction, allowing synthesis of an antigenic component which has an intramolecularly formed disulfide bond to sustain. Dithiothreitol (hereinafter, this may be referred to as "DTT") as a reducing agent is decreased to have a final concentration of 20 to 70 µM, and preferably 30 to 50 µM in the final solution for translation reaction prepared from a wheat embryo extract-containing solution. 2-mercaptoethanol is decreased to have a final concentration of 0.1 to 0.2 mM in the final solution for translation reaction. Glutathione/oxidized glutathione is decreased to have a final concentration of 30-50 µM/1-5 µM in the final solution for translation reaction. The specific concentration of a reducing agent is not limited to those described above and varies appropriately depending on the protein to synthesize or the kind of a cell-free protein synthesis system to use.

The method of selecting the optimal concentration range of a reducing agent is not limited in particular, and, for example, there is mentioned a selection method based on the effect of a catalyst for disulfide bond exchange reaction. Specifically, solutions for translation reaction are prepared from a wheat embryo extract-containing solution at various concentrations of a reducing agent, and then supplied with a enzyme capable of catalyzing disulfide bond exchange reaction to synthesize an antigenic component having an intramolecular disulfide bond. In addition, as a control experiment, the same protein synthesis is carried out using the same solutions for translation reaction supplied with no enzyme capable of catalyzing disulfide bond exchange reaction. Then, the soluble component of an antigenic component to synthesize is separated by a method such as centrifugation. The reaction solution, wherein this soluble component has a share of 50% (solubilization 50%) or more in the total and further has been increased by the addition of an enzyme capable of catalyzing disulfide bond exchange reaction, can be determined to be a suitable reaction solution for synthesizing an antigenic component with an intramolecular disulfide bond retained. Furthermore, within the concentration range of a reducing agent selected based on the effect of the catalyst for disulfide bond exchange reaction as above described, the concentration of the reducing agent which can synthesize the highest amount of the antigenic component can be selected as more preferable concentration range.

For specific methods for decreasing a reducing agent, there is used a method wherein a wheat embryo extract-containing solution is prepared to be free from a reducing agent, and then supplied with a reducing agent to have an above described concentration range together with necessary components for a cell-free protein synthesis system, or a method wherein a reducing agent is removed from a solution for translation reaction derived from a wheat embryo extract-containing solution to be within the concentration range described above. As a wheat embryo extract-containing solution for cell-free protein synthesis requires a high degree of reduction condition to extract, a method wherein a reducing agent is removed from the solution after extraction is easier to execute. As a method for removing reducing agent from a wheat embryo extract-containing solution, there is mentioned a method using a gelfiltration support. Specifically, for example, there is mentioned a method wherein Sephadex G-25 column is beforehand equilibrated with an appropriate buffer containing no reducing agent, and then fed with a wheat embryo extract-containing solution to pass through.

(6) Preparation of Solution for Translation Reaction
The wheat embryo extract-containing solution prepared as described above is supplied with a nuclease inhibitor, various ions, a substrate, an energy source and the like necessary for protein synthesis (hereinafter, they may be referred to as "additives for a solution for translation reaction") and a mRNA encoding a target antigenic component, which acts as a translation template, and, if desired, a stabilizer which comprising a component selected from the group consisting of inositol, trehalose, mannitol, and sucrose-epichlorohydrin copolymer to prepare a solution for translation reaction. The concentrations of components to add may be provided from a compounding ratio well known per se.

The additives for a solution for translation reaction, specifically, include amino acids acting as substrate, an energy source, various ions, a buffer, an ATP-regenerating system, a nuclease inhibitor, a tRNA, a reducing agent, polyethylene glycol, a 3', 5'-cAMP, a folate, an antimicrobial, and the like. Further, concerning each concentration, preferably, ATP is contained at 100 µM to 0.5 mM, GTP at 25 µM to 1 mM and 20 kinds of amino acids at their respective 25 µM to 5 mM. They can be selected and combined for use as appropriate according to the translation reaction system. Specifically, a wheat embryo extract, which is used for a wheat embryo extract-containing solution, is supplied with 20 mM of HEPES-KOH (pH 7.6), 100 mM of potassium acetate, 2.65 mM of magnesium acetate, 0.380 mM of spermidine (from Nacalai Tesque), respectively 0. 3 mM of 20 kinds of L-amino acids, 4 mM of dithiothreitoll, 1.2 mM of ATP (from Wako Pure Chemical Industries, Ltd.), 0.25 mM of GTP (from Wako Pure Chemical Industries, Ltd.), 16 mM of phosphocreatine (from Wako Pure Chemical Industries, Ltd.), 1000U/ml of Rnase inhibiter (from TAKARA) and 400µg/ml of creatine kinase (from Roche), to dissolve sufficiently, followed by adding the mRNA translation template supporting a mRNA encoding a target antigenic component.

Herein, the mRNA encoding the target antigenic component has a structure wherein the sequence encoding an antigenic component capable of being synthesized in a cell-free protein synthesis system with a wheat embryo is linked in the downstream of both an appropriate sequence recognized by RNA polymerase and further a sequence having a function to activate translation. The sequence recognized by RNA polymerase includes T3 or T7 RNA polymerase promoter, or the other. Further, in a cell-free protein synthesis system, as a sequence enhancing a translation activity, there may be preferably used a sequence having a structure wherein Q sequence, Sp6 or the other is linked to the 5'-upstream of the coding sequence.

(7) Malaria Antigen
For the present invention, based on genome information (Gardner et al.: Genome sequence of the human malaria parasite Plasmodium falciparum. Nature 419: 498-511, 2002), the genome of Plasmodium falciparum (of 22.9Mbp in size and composed of 5268 genes) can preferably be used. Approximately sixty percent of proteins encoded by them were not similar to any proteins previously known, thereby even their functions were not predictable, so their function has not been analyzed so far. Moreover, for a protein from Plasmodium falciparum, the exon region has an AT content of as high as 76% on average, and hence the expression of the recombinant protein is difficult in conventional systems.
In the present invention, the genome of vivax malaria can also preferably be used. In particular, genes of Pvs25 and Pvs28 used in the development of vaccines for preventing vivax malaria propagation also can be used (T.Tsuboi, D.C. Kaslow, M.M.G. Gozar, M. Tachibana, Y-M. Cao, M. Torii:Sequence polymorphism in two novel Plasmodium vivax ookinete surface proteins, Pvs25 andPvs28, that are malaria transmission-blocking vaccine candidates, Mol. Med. 4,772-782, 1998). Although expressing a recombinant protein in a yeast using these genes, then immunizing an animal, and inducing an effective antibody for preventing propagation by that recombinant protein has been found feasible (H. Hisaeda, A.W. Stowers, T. Tsuboi, W.E. Collins, J. Sattabongkot, N.Suwanabun, M. Torii, D.C. Kaslow: Antibodies to malaria vaccine candidatesPvs25 and Pvs28 completely block the ability of Plasmodium vivax to infect mosquitoes. Infect. Immun. 68, 6618-6623, 2000) (T. Arakawa, T. Tsuboi, A.Kishimoto, J. Sattabongkot, N. Suwanabun, T. Rungruang, Y. Matsumoto, N. Tsuji, : H.Hisaeda, A. Stowers, I. Shimabukuro, Y. Sato, M. Torii: Serum antibodies induced by intranasal immunization of mice with plasmodium vivax Pvs25 co-administered with cholera toxin completely block parasite transmission to mosquitoes. Vaccine21: 3143-3148, 2003), the cell-free synthesis system of the present invention also induced an effective antibody for preventing propagation likewise.

Concerning the antigenic component of the present invention, previously known malaria antigens as described above and new antigenic components prepared by the means of the present invention can be a subject of the present invention. In the present invention, as an antigenic component can be prepared in a cell-free synthesis system without depending on codon usage, the site of the desired base sequence based on the genome information is selected without a conversion corresponding to a codon usage and directly subjected to transcription/translation through the synthesis system of the present invention, allowing easy production of an antigenic component with a almost native state of conformation. Furthermore, a useful antigenic component can be easily screened by immunizing with the obtained antigenic component as an immunogenic substance, and then determining the reactivity of the prepared antiserum with the target microbe or a substance derived from the microbe.
For example, transcriptions are conducted on the basis of the base sequences of SEQ. ID. Nos: 1, 3, 5, and 7, and the mRNAs as translation templates are produced and used to produce antigenic components through the cell-free synthesis. The polypeptides thus obtained are used to immunize. This example is disclosed just by way of a preferable illustration and not by way of limitation. The amino acid sequences of antigens shown in the Examples are represented by SEQ. ID. Nos: 2, 4, 6 and 8.
Meanwhile, an antigenic component with the retained antigenicity according to the present invention does not only mean a protein which is identical to or has a homology of 70% or more in an amino acid sequence to a native antigenic component synthesized through protein synthesis system, but also a protein which is recognized by the antibody just like a native antigenic component. Its conformation is thought to be similar to a native protein.

(8) Method of Synthesizing Protein Using Cell Extract Solution for Cell-Free Protein Synthesis
The wheat embryo extract solution, that is, a cell extract solution prepared as above for cell-free protein synthesis can be dissolved in a dissolving solution which is supplied with a deliquescent substance and water to have a concentration appropriate for protein synthesis reaction, and put into a selected system or apparatus already known per se to take place protein synthesis. As a system or apparatus for protein synthesis, there are mentioned a method such as the batch method (Pratt, J. M. et al., Transcription and Tranlation, Hames, 179-209; B. D. & Higgins, S. J., eds, IRL Press, Oxford (1984)) wherein a translation reaction solution in which the cell extract solution for cell-free protein synthesis of the present invention is dissolved is kept at an appropriate temperature for the synthesis, a cell-free protein synthesis system in a continuous manner (Spirin, A. S. et al., Science, 242, 1162-1164 (1988)) wherein amino acids, an energy source and others necessary for a cell-free protein synthesis system are fed into the reaction system continually, a dialysis method (Kigawa et al., The 21st The Molecular Biology Society of Japan, WID6), and a method wherein a solution containing amino acids, an energy source and others necessary for a cell-free protein synthesis system is overlaid onto a solution for translation reaction (bilayer system: Sawasaki, T., et al., FEBS LETTERS 514, 102-105 (2002)).

Herein, when the cell extract for cell-free protein synthesis is used at a decreased concentration of a reducing agent, a solution for supplying amino acids, an energy source and others necessary for a cell-free protein synthesis system also is adjusted to have the same concentration of the reducing agent. Furthermore, the translation reaction is conducted in the presence of an enzyme capable of catalyzing disulfide bond exchange reaction to allow high efficient synthesis of an antigenic component which retains an intramolecular disulfide bond. As the enzyme capable of catalyzing disulfide bond exchange reaction, for example, a protein disulphide isomerase may be mentioned. The amount of these enzymes to add to a cell-free translation system described above may be selected as appropriate depending on the kind of enzyme. Specifically, the solution for translation reaction, that is, a wheat embryo extract-containing solution which is extracted from wheat embryo and contains DTT as a reducing agent at 20 to 70 µM and preferably 30 to 50 µM is supplied with a protein disulfide isomerase to have a final concentration of 0.01 and 10 µM, and preferably 0.5 µM in the reaction solution for translation. Further, the addition is preferable in timing before the initiation of translation reaction in view of the efficiency of the formation of a disulfide bond.

### (Method for Preparing Antigenic Component)

Malaria antigen shown as an example of the present invention is presented as a region showing important biological functions based on the previously known sequence described above (like, for example, polypeptides represented by SEQ. ID. Nos: 2, 4, 6, and 8). The amino acid composing the antigenic component is not necessarily identical to the amino acid sequence of a previously known malaria antigen, but as long as it has functions as an immunogen similar to those of the polypeptide, it may be a polypeptide wherein a mutation such as deletion, substitution, addition, and insertion is introduced into the amino acid sequence of the polypeptide as appropriate, and also it may be an amino acid sequence having a homology of 70% or more to that. The antigen like this can easily be prepared by any and all methods widely known as a purification method of polypeptides or proteins. If a useful antigenic component is identified to be useful, it can easily be recovered by tagging the recombinant protein with a well known tag to purify through the affinity to the tag, or by using an antibody to the antigen to carry out affinity chromatography.
In the present invention useful antigenic components are not necessarily limited to the sequences described above. The application of the screening approach of the present invention allows to provide a novel antigenic component easily.
Further, the person skilled in the art could identify an epitope portion by a well known screening approach, synthesize at least 3 or more, preferably from 5 to 15 polypeptides as appropriate, and then use them as antigenic components.

### (Preparation of Antibody)

In the present invention, the prepared antigenic component as described above is used to prepare antibodies which immunologically recognize it. The antibodies are those which respond to polypeptides represented by, for example, SEQ. ID. Nos: 2, 4, 6 and 8. However, as the antigenic component obtained by the present invention can be constructed to have a conformation similar to the native, this invention is not be limited to the antibodies. Namely, antibodies corresponding to any and all antigenic components prepared by the method of the present inventionn are also subject of this invention.
An antibody is prepared alone or in linking to a support by an antigenic polypeptide in the presence or absence of an adjuvant through the induction by cellular and/or humoral responses. While an antigenic polypeptide such as an antigenic component comprising the amino acid sequence represented by SEQ. ID. No: 2, 4, 6 or 8 may be directly used as an immunogen, the antigenic polypeptide may be used to design another antigenic polypeptide. Usually, a fragment consisting of as much as 5 amino acids characterizes an antigenic region, and hence an antigenic polypeptide, for example, is thought to have a sequence which consists of at least 5 amino acids and centers the sequence represented by SEQ. ID. Nos: 2, 4, 6 and 8. The polypeptide, which is too small to have a sufficient antigenicity, may be linked to a suitable support.
To obtain such a linkage, many methods are widely known in the field of interest, including a method for forming a disulfide bond using N-succinimidyl-3-(2-pyridylthio)propionate (SPDP) and succinimidyl 4-(N-maleimidemethyl)cyclohexane-1-carboxylate(SMCC) supplied by Pierce Company, Rockford, Illinois [see, for example, Immun. Rev. (1982) 62: 185]. As the support, any support may be used as long as it does not induce the production of an antibody detrimental to the host. Suitable supports include, typically, protein, polysaccharide, polymerized amino acid, amino acid copolymer and inert viral particle. Particularly useful proteins include serum albumin, Keyhole Limpet Hemocyanin, immunoglobulin molecule, thyroglobulin, egg albumin, tetanus toxin and other proteins widely known to the person skilled in the art.

The prepared antigenic component is used to produce both polyclonal and monoclonal antibodies. If a polyclonal antibody is desired, a mammal selected (for example, mouse, rabbit, goat, horse, etc.) is immunized with the antigenic component. A blood serum obtained from the immunized animal is recovered to treat by a widely known method. If a blood serum containing a polyclonal antibody also contains an antibody to the other antigen than the desired, this polyclonal antibody may be purified by immunoaffinity chromatography. A method for producing and processing a polyclonal antiserum is widely known in the field of interest. The example is a method described in Mayer and Walker (1987): IMMUNOCHEMICAL METHODS INCELLAND MOLECULAR BIOLOGY (Academic Press, London).
A monoclonal antibody may also be produced easily by the person skilled in the art. A general method for preparing a monoclonal antibody with a hybridoma is widely known. A permanently proliferative antibody-producing cell system can be prepared by cell fusion. Further, it can also be prepared by other methods such as direct transformation of a B lymphocyte using a tumorigenic DNA, or transfection using Epstein-Barr virus. Examples are the methods described in J. Virol. 60: 1153. Schreier, M. et al. (1980); Virology 162: 167. Hammerling et al. (1981); British Medical J. 295: 946. Kennett et al. (1980). Further, to make them usable for diagnosing and treating human diseases, the humanization of these monoclonal antibodies is also included.

Both of the formed monoclonal and polyclonal antibodies can recognize a part of the sequence of antigenic component to be excellent in antigen-antibody reactivity. Then, the usability of the obtained antibody can be determined by confirming its reactivity to the target microbe or a protein derived therefrom. The antibody obtained by the method of the present invention has a high immunoreactivity as can be seen in experimental Examples. Therefore, an antibody obtained by the method of the present invention is useful as an antibody to a microbe, for example, anti-plasmodium antibody (hereinafter, often referred to as malaria antibody).

Further, monoclonal antibodies are prepared and screened for an antibody usable as a malaria antibody, allowing selection of a useful antigenic component. Based on the epitope information, the epitope portion is selected, allowing easier production of a low molecular vaccine.

The vaccine according to the present invention can be provided in the form of a pharmaceutical composition in combination with, for example, a pharmaceutically acceptable carrier or adjuvant. A pharmaceutically acceptable carrier for use in the present invention is the one suitable for the case in which immunocompetent cells of a living body recognize a vaccine antigen. Further, an immunization adjuvant for use in the preparation of pharmaceutical composition is well known in the art. The person skilled in the art can select an adequate adjuvant as appropriate to produce a pharmaceutical composition. The vaccine of the present invention can be used in any administration forms, such as capsule, suspension, elixir or solution. Furthermore, in preparing a pharmaceutical composition comprising a vaccine of a single application dose, the amount of the protein to be combined with a carrier substance usually depends on many factors including the route and method of administration, the stability and activity (immunogenicity) of the antigenic protein, the sex, age, weight, and general health of the subject, viral pathogen's type to prevent or treat, and the like. The amount of vaccine to administer also depends on the kind and amount of a pharmaceutically acceptable carrier and adjuvant.

The diagnostic agent comprising the antibody according to the present invention can be put in use with the antibody linked to an immobilization support or labeling substance, but is not limited in particular. As an immobilization support, polystyrene resin, acrylamide resin, latex particle, gelatine particle and the like may be mentioned. As a labeling substance, enzymes (for example, peroxidase, β-D-galactosidase; by enzyme immunoassay), Fluorescence emission substances (for example, FITC; by fluoroimmunoassay), radio-isotopes (for example, ¹²⁵I; by radioimmunoassay), chemiluminescent substances (for example, luminol; by chemiluminescent immunoassay), gold colloid and the like may be mentioned. Meanwhile, in order to link an antibody according to the present invention with an immobilization support or labeling substance, a spacer construct may be intervened between them by a covalent bond. Further, for assay, any and all immunoassays such as enzyme immunoassay, fluoroimmunoassay, radioimmunoassay, chemiluminescent immunoassay, particle agglutination, immunochromatography can be used. Furthermore, a diagnostic kit comprising the diagnostic agent indicated above can be provided as a rapid and easy diagnostic tool.

A method for screening an antigenic component using a cell-free protein synthesis means with the use of a wheat embryo extract according to the present invention comprises the following steps:
1) Preparing a gene comprising a selected region of a desired candidate gene.
   A candidate gene region is selected on the basis of the genome information. Herein, in a cell-free protein synthesis system with the use of a wheat embryo extract of the present invention, even the AT rich region of a selected candidate gene can be a subject because the AT content of a gene encoding a protein to synthesize will not affect protein synthesis. Furthermore, a tag sequence necessary for purification of synthesized polypeptide may be comprised.
2) Synthesizing a mRNA from the gene prepared in step 1) .
   Transcription can be conducted by a conventionally known method.
3) Synthesizing a polypeptide by a cell-free protein synthesis means with the use of a wheat embryo extract through translation using the mRNA synthesized in step 2) as a translation template. Alternatively, a polypeptide is synthesized by a cell-free protein synthesis system in an integrated transcription/translation manner using the gene synthesized in step 1) as a template.
   The cell extract solution for cell-free protein synthesis with a wheat embryo extract is added into a container. Subsequently, a solution comprising substances necessary for polypeptide synthesis, a translation or transcription template, and a stabilizer are added to synthesize a polypeptide. Synthesis can be conducted by putting the cell extract solution into the different wells of the container divided into a plurality of regions with, for example, Pipetman and/or the channel pipette of an automatic pipetting device. In this instance, the cell extract solution for cell-free protein synthesis with a wheat embryo extract is added in an amount appropriate to the well volume, and then the necessary amount of a solution comprising substances necessary for polypeptide synthesis, a translation or transcription template, and a stabilizer are added into each well with, for example, Pipetman and/or the channel pipette of a automatic pipetting device to synthesize a polypeptide.
4) Immunizing an individual mammal with the polypeptide synthesized in step 3) indicated above to obtain an antiserum.
   Mammals (for example, mouse, rabbit, goat, horse, etc) are immunized with the polypeptide synthesized in step 3) by a conventionally known method. The blood serums obtained from the immunized animals are recovered and treated by a method widely known. If the blood serum comprising a polyclonal antibody contains an antibody to the other antigen than the desired, this polyclonal antibody may be purified by immunoaffinity chromatography. A method for producing and processing a polyclonal antiserum is widely known in the field of interest. Furthermore, a monoclonal antibody may also be easily produced by the person skilled in the art.
5) Reacting a desired target microbe or a protein derived therefrom with the antiserum described above to analyze their reactivity.
   The antiserum obtained in step 4) is tested to determine whether it reacts with the desired target microbe, for example, protozoa. The antiserum obtained in step 4) can also be tested to determine whether it reacts with a protein derived from the microbe. For these reactions, secondary antibodies such as fluorescently labeled antibody may be used. In that case, the fluorescence of the secondary antibody is confirmed by microscopic observation and the like.
6) Identifying the selected region of a candidate gene from which the antiserum has been derived with its confirmed reactivity as a candidate antigenic component.
   The polypeptide used to make an antiserum whose reactivity is confirmed by, for example, detecting fluorescence in step 5) may be referred to as an antigenic component with the retained antigenicity.

The present invention is explained in detail below with reference to Examples, but these examples are not intended to limit the scope of the present invention.

### EXAMPLE 1

### Cell-Free Protein Synthesis

### (1)Preparation of Wheat Embryo Extract Solution

The seeds of Chihoku wheat produced in Hokkaido or those of Chikugoizumi produced in Ehime were fed into a mill (from Fritsch: Rotor Speed Millpulverisette Type 14) at the rate of 100g/min. and pulverized gently at a speed of 8,000 rpm. After collecting a fraction containing a wheat embryo having germinability by a sieve (sieve opening from 0.7 to 1. 00 mm), selection by flotation with the mixture of carbon tetrachloride and cyclohexane (volume ratio; carbon tetrachloride: cyclohexane = 2.4:1) was conducted to recover a floating fraction containing a wheat embryo having germinability, then organic solvent medium was dried off at room temperature, and then mixed impurities such as seed coats were removed by blowing at room temperature to obtain a crude wheat embryo fraction.
Next, using a belt type color sorter, BLM-300K (manufacturer: Anzai Manufacturing Co., Ltd., distributor: Anzai Corporation, Ltd.), a wheat embryo was sorted out from a crude wheat embryo fraction by taking advantage of differences in color as below. This color sorter is an apparatus that comprises a means for irradiating light to the crude wheat embryo fraction, a means for detecting reflected and/or transmitted beams from crude wheat embryo fraction, a means for comparing the detected value with a reference value, and a means for sorting them into classes without and within the scope of the reference value.
A crude wheat embryo fraction was fed onto the beige-colored belt of the color sorter at a rate of 1000 to 5000 grains/cm², then the crude wheat embryo fraction on the belt was irradiated with the light of a fluorescent lamp and its reflected light detected. The belt was set to convey at a speed of 50m/min. As a photosensor, a monochrome CCD line sensor (2048 pixels) was used.
First, to eliminate darker color components than a wheat embryo (seed coat etc.), a reference value was set between the wheat embryo luminance and the seed coat luminance, and the component exceeding the reference value was sucked to eliminate. Then, to sort out an endosperm, a reference value was set between the wheat embryo luminance and the endosperm luminance, and the component exceeding the reference value was sucked to eliminate. 30 suction nozzles (1 suction nozzle per 1cm length) were placed at the position approximately 1cm above the conveyer belt to suck.
This method was repeated to sort the wheat embryo until it has a wheat embryo purity (a weight ratio of wheat embryo contained per 1g of random sample) of 98% or higher.
The obtained wheat embryo fraction was suspended in distilled water at 4 °C, and washed with a rinsing solution in an ultrasonic cleaner until the solution got free from white turbidity. Then, it was suspended in 0.5 v% solution of Nonidet (from Nacalai Techtonics) P40, and washed with a rinsing solution in the ultrasonic cleaner until the solution got free from white turbidity to obtain wheat embryo, and then the following operations were conducted at 4 °C.
An extractant (80 mM of HEPES-KOH, pH 7.8, 200 mM of potassium acetate, 10 mM of magnesium acetate, 8 mM of dithiothreitol, (each 0.6 mM of 20 kinds of L-amino acids may have been added)) of twice the volume of wet weight of the washed wheat embryo was added, and then the wheat embryo was limitedly homogenized 3 times at 5,000 to 20,000 rpm for 30 seconds using a Waring blender. This homogenate was centrifuged at 30, 000 x g for 30 minutes using a high-speed centrifuge to give a supernatant, which was centrifuged again in a similar condition to obtain a supernatant. This sample was subjected to long-term storage at -80 °C or below, resulting in no deterioration of the activity. The obtained supernatant was filtered with a filter having a pore size of 0.2µm (NEW Steradisc 25: supplied by Kurabo Industries Ltd.) to sterilize by filtration and eliminate contaminating fine dusts.
Next, this filtrate was subjected to gelfiltration using Sephadex G-25 column which had been equilibrated with a solution [40 mM of HEPES-KOH (pH 7.8), and the mixture of, respectively, 100 mM of potassium acetate, 5 mM of magnesium acetate, 8 mM of dithiothreitol, each 0.3 mM of 20 kinds of L-amino acids (amino acids may be present, absent or labeled depending on the purpose of protein synthesis)] in advance. The obtained filtrate was centrifuged again at 30,000 x g for 30 minutes to recover a supernatant, which was adjusted to have a concentration of 90 to 150 at A260nm (A260/A280=1.4 to 1.6).
To the obtained wheat embryo extract-containing solution for protein synthesis, 20 mM of HEPES-KOH (pH 7.6), 100 mM of potassium acetate, 2. 65 mM of magnesium acetate, 0. 380 mM of spermidine (from Nacalai Techtonics), each 0.3 mM of 20 kinds of L-amino acids, 4 mM of dithiothreitol, 1.2 mM of ATP (from Wako Pure Chemical Industries, Ltd.), 0.25 mM of GTP (from Wako Pure Chemical Industries, Ltd.), 16 mM of phosphocreatine (from Wako Pure Chemical Industries, Ltd.), 1000U/ml of Rnaseinhibitor (from TAKARA), 400µg/ml of creatine kinase (from Roche) were added to prepare the source of solution for translation reaction.

### (2) Preparation of Transcription Template and Translation

In the preparation of a transcription template, the genes of SEQ. ID. Nos: 1, 3, 5 and 7 were used to clone each the gene in EcoRV site of multiple cloning sites of a plasmid, pEU3-NII (SP6), (Proc Natl Acad Sci USA,2002, vol 99, p14652-14657 : Sawasaki,T et al.) suitable for a wheat embryo cell-free protein synthesis system. In the present invention, unaltered base sequences derived from protozoa were used without having been optimized by a codon usage. A pEU3NII (SP6) vector integrated with the following genes: Pfs25-TBV (which was cloned by the present inventor Tsuboi from a plasmid supplied by Invitrogen comprising Pfs25-TBV sequence for use), Pfs25-3D7 (which was cloned by the present inventor Tsuboi from the genome DNA of Plasmodium falciparum for use), Pvs25 (which was cloned by the present inventor Tsuboi from the genome DNA of Plasmodium vivax for use) and Pvs28 (which was cloned by the present inventor Tsuboi from the genome DNA of Plasmodium vivax for use) was used as a transcription template to transcript in 1 ml of the system at 37 °C for 3 hours. The whole pellet of the obtained mRNA was added to 1 ml of the wheat embryo extract solution (60 O.D.) indicated above in (1) and protein synthesis was conducted at 26 °C for 48 hours.

Referring to the purification of an antigenic component, the buffer in 450 µl of the obtained reaction solution for protein synthesis was exchanged by MicroSpin G-25 Column (from Amersham Biosciences), and the solution was subjected to 250 µl of Ni-NTA Superflow (from QIAGEN). After washing the resin, a cell-free synthesis protein was eluted in 200 mM of imidazol, and the eluate was subjected to Superdex 75 pg (from Amersham Pharmacia Biotech) to obtain a purified protein.

### a. Gene Specification:

### Pfs25-3D7 (Nature. 1988 May 5;333 (6168):74-6)

Pfs25 is a kind of surface proteins of ookinete, which is a developing stage of Plasmodium falciparum in the midgut of a mosquito. 3D7 is a name of the cultured strain of cloned Plasmodium falciparum of this gene. A site called EGF-like domain (four repeats of an analogous domain) with both a hydrophobic N-terminal signal sequence and a C-terminal GPI anchor signal deleted from Pfs25 was used, amplification was conducted by PCR using the genome DNA of 3D7 cultured strain of Plasmodium falciparum as a template, a sense primer (SEQ. ID. No: 9) and an antisense primer (SEQ. ID. No: 10). There were totally twenty-two cysteine residues comprised in the sequence. The sequence used for the expression is represented by SEQ. ID. No: 1 (which has an artificially inserted start codon, a six consecutive histidine tag codon and a stop codon, is composed of 561 bases, and has an AT content of 70.2% for the sites excluding His-tag codon) in the sequence listing. Further, the amino acid sequence encoded is represented by SEQ. ID. No: 2 in the sequence listing (186 residues).

### Pfs25-TBV [Biotechnology (N Y). 1994 May; 12(5):494-9]

This antigen is an artificially synthesized antigen from the above described Pfs25 gene which has an codon changed to be appropriate for expression in an yeast. Currently, as a vaccine antigen for preventing the propagation of falciparum malaria, the vaccine for the first phase clinical trial is being manufactured through expression using an yeast (Pichiapastoris) by the malaria vaccine developing department of NIH in the United State. At the three N-glycosylation sites, which would interfere the expression of recombinant proteins in an yeast, asparagines (N) are replaced by glutamines (Q) and a six consecutive histidine-tag is added to C-terminal end. The construct for a cell-free protein synthesis system was amplified by PCR using an artificially synthesized DNA offered by NIH as a template, a sense primer (SEQ. ID. No: 11), and an antisense primer (SEQ. ID. No: 12). The sequence used for the expression is represented by SEQ. ID. No: 3 in the sequence listing (which has an artificially inserted start codon, a six consecutive histidine-tag codon and a stop codon, is composed of 540 bases, and has an AT content of 58. 4% for the sites excluding the His-tag codon) . Further the amino acid sequence encoded by the sequence is represented by SEQ. ID. No: 4 in the sequence listing (179 residues).

### Pvs25 (Mol. Med. 4, 772-782, 1998;Infect. Immun. 68, 6618-6623, 2000)

Pvs25 is a kind of surface protein of ookinete, which is the developing stage of Plasmodium vivax in the midgut of a mosquito. A site called EGF-like domain (four repeats of an analogous domain) with both a hydrophobic N-terminal signal sequence and C-terminal GPI anchor signal deleted was used for expression, and amplification was conducted by PCR using the genome DNA of Plasmodium vivax SalvadorI strain as a template, a sense primer (SEQ. ID. No: 13), and an antisense primer (SEQ. ID. No: 14). There were totally included twenty-two cysteine residues. The sequence used for the expression is represented by SEQ. ID. No: 5 in the sequence listing (which has an artificially inserted start codon, a six consecutive histidine-tag codon and a stop codon, is composed of 555 bases, and has an AT content of 57.5% for the sites excluding the His-tag codon). Further, the amino acid sequence encoded by the sequence is represented by SEQ. ID. No: 6 in the sequence listing (184 residues).

### Pvs28 (Mol. Med. 4, 772-782, 1998;Infect. Immun. 68, 6618-6623, 2000)

Pvs28 is also a kind of surface protein of ookinete, which is the developing stage of Plasmodium vivax in the midgut of a mosquito. For expression, a site called EGF-like domain (four repeats of an analogous domain) with both a hydrophobic N-terminal signal sequence and a C-terminal GPI anchor signal deleted and a repeat sequence by six repetitions of the amino acids GSGGE/D added to exist was used. Among Pvs28 genes cloned from Plasmodium vivax SalvadorI strain in NIH, there was used a DNA wherein asparagine (N) was replaced by glutamine (Q) at the N-glycosylation site of the 130th residue from the N-terminus. Amplification was conducted by PCR using the DNA as a template, a sense primer (SEQ. ID. No: 15), and an antisense primer (SEQ. ID. No: 16). There were totally included twenty cysteine residues. The sequence used for the expression is represented by SEQ. ID. No: 7 in the sequence listing (which has an artificially inserted start codon, a six consecutive histidine-tag codon and a stop codon, is composed of 612 bases, and has an AT content of 52.6% for the sites excluding the His-tag codon). Further, the amino acid sequence encoded by the sequence is represented by SEQ. ID. No: 8 in the sequence listing (203 residues).

### EXAMPLE 2

### Confirmation of Expression of Antigen Component

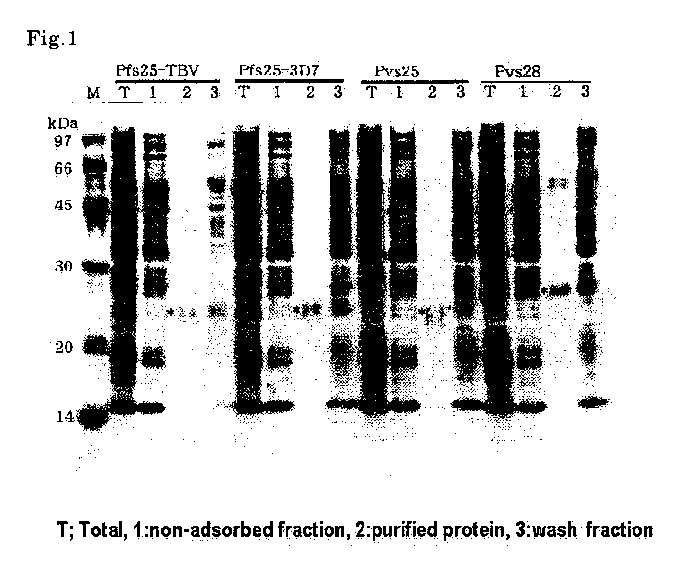

Pfs25-TBV, Pfs25-3D7, Pvs25 and Pvs28 after the protein synthesis in Example 1 was subjected to purification by His-tag. The obtained samples were subjected to SDS-PAGE using 12.5% polyacrylamide gel under a reductive condition and stained with CBB (Fig. 1). 0.5 µl of a reaction solution for protein synthesis was added to the total lane, and the samples of approximately 3-fold the volume of the solution were added to the other lanes for electrophoresis. As a result, all four kinds of proteins were confirmed to be expressed to have almost aimed molecular sizes (marked * in Fig. 1) by almost same amounts. It should be noted that Pfs25-3D7 which used the original codon of Plasmodium falciparum and had an AT content of 70.2% was confirmed to provide an almost same amount of protein as Pfs25-TBV which was an artificially synthesized gene having an AT content of 58.4%. This result suggests that the present invention is extremely useful for genes having an average AT content of 76% for all the genes to express the proteins of Plasmodium falciparum, which cannot be provided by an existing method for expressing recombinant proteins. Further, the significant difference in amount of a protein expressed was not seen between the presence and absence of an N-glycosylation site. (Pfs25-TBV: no site, Pfs25-3D7: three sites).

### EXAMPLE 3

### Immunostaining of Plasmodium with blood serum (anti-Pvs25 or anti-Pvs28 mouse blood serum, anti-Pfs25-3D7 or anti-Pfs25-TBV mouse blood serum)

The purified proteins obtained in Examples 1 and 2 were respectively adjusted to have a concentration of 10µg/50 µl PBS, emulsified together with 75 µl of Freund's complete adjuvant (Wako Pure Chemical Industries, Ltd.), and intraperitoneally administered to an 8-week old BALB/c female mouse (CLEA Japan, Inc.). Two mice were allocated to each group, and the negative control group was immunized likewise with a protein (FT, His-tagged) derived from vegetable made in the same manner as described above in a cell-free protein synthesis system. Three weeks after the first immunization, an additional immunization was conducted using Freund's incomplete adjuvant (Wako Pure Chemical Industries, Ltd.), thereafter totally three additional immunizations were carried out every two weeks. Two weeks after the fourth immunization in total, the whole bloods were collected from the hearts under etherization. After the collection of blood, the bloods were left to stand at room temperature for 1 hour, then at 4 °C overnight, and the blood sera were separated the next day. The separated blood sera were frozen to store at -80 °C until used for experiments.

### (Test Method)

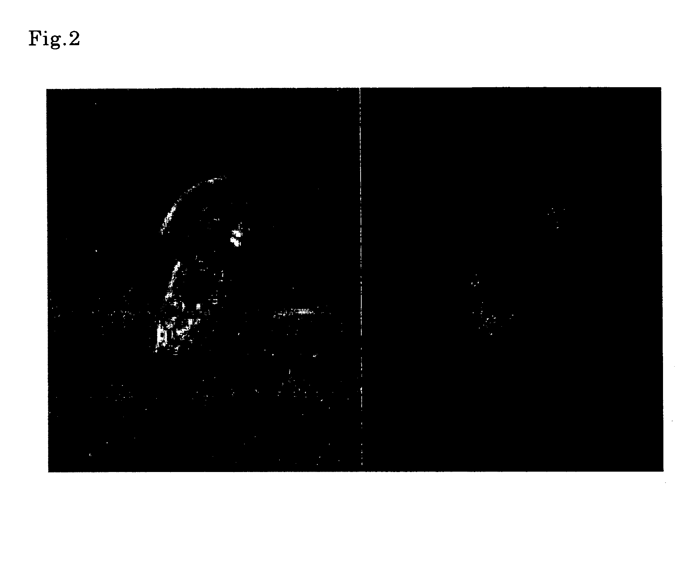

The gametocyte of Plasmodium vivax from a patient living in vivax malaria epidemic region in Thailand was purified and cultured using an ookinete culture solution at 26 °C overnight to obtain an ookinete. A cultured protozoa was spotted onto a slide glass and immobilized with acetone. Then anti-Pvs25 or anti-Pvs28 mouse blood serum indicated above was used as a primary antibody, FITC-labeled antimouse antibody was used as a secondary antibody, and DAPI was used for nuclear staining. The stained specimens were observed with NikonC1 confocuslaser scanning microscope. Further, Plasmodium falciparum ookinete made likewise using the gametocyte of a falciparum malarial patient was used as an antigen, and anti-Pfs25-3D7 or anti-Pfs25-TBV mouse blood serum indicated above was used to stain in the same manner as described above. Fig. 2 shows Plasmodium vivax ookinete stained with anti-Pvs28 mouse blood serum as representative's example.

### (Test Result)

Previous researches have revealed that the four kinds of protein described above are expressed on the surface of plasmodium ookinete. The most important was that an antibody which recognized the conformation of the protein of protozoa was essential for demonstrating the fact. For example, when Pfs25 recombinant protein was expressed in E. Coli and immunized an animal, an antibody to this recombinant protein was indeed produced, but this antibody was not able to react with a protozoa's protein.

Whether antibodies to the four kinds of above-described proteins synthesized using a wheat embryo-using cell-free protein synthesis system can react with a protozoa' s protein or not is the most important point to determine how much these proteins used as antigens resemble in conformation the original protein of the protozoa. If an ookinete surface protein which has been the most difficult to express by existing systems is identified to have antigenicity, it would be the most useful for determining the meaning of the present invention.

Fig. 2 shows that Plasmodium vivax ookinete made by culturing from a patient blood was stained with a mouse antiserum to Pvs28 made through a cell-free protein synthesis system. The left is a photograph of protozoas and surrounding erythrocytes (7 µm in diameter) taken by a light microscope. The right photograph is the photofluorographic tomogram of protozoa-thick midsection by laser-scanning the same visual field. Green stains show the localization of Pvs28 in protozoa and blue ones protozoa's nuclei. This result shows that anti-Pvs28 mouse blood serum contains an antibody which can link to original Pvs28 protein of a protozoa, and Pvs28 is expressed on an ookinete surface. Likewise, Pvs25 antiserum can stain Plasmodium vivax ookinete, and anti-Pfs25-TBV and anti-Pfs25-3D7 antiserums can stain Plasmodium falciparum ookinete. Therefore, from these results, it is demonstrated that the wheat embryo cell-free protein synthesis system allows production of four kinds of proteins as vaccine candidate antigens for preventing the propagation of malaria so that they may have similar conformations to the original structure of a protozoa.

### EXAMPLE 4

### Confirmation of Activity of Anti-Pvs25 or Pvs28 Mouse Blood Serum for Preventing Propagation of Malaria

From a vivax malarial patient who visited a malaria clinic, managed by the government of Thailand, located on the border between Thailand and Myanmar, a protozoa-infected blood was collected under the patient's consent, divide into every 300 µl, and freed from the patient's own plasma, followed by adding various different blood sera to the infected erythrocytes under the condition described below to reconstruct their respective bloods, which malaria mediator mosquitos (Anophelesdirus) in Thailand were then allowed to suck for 30 minutes using a artificial membrane blood sucking apparatus.

1. 150 µl of healthy type AB blood serum was added.
2. 75 µl of healthy type AB blood serum plus 75 µl of anti-FT mouse blood serum (negative control) was added.
3. 75 µl of healthy type AB blood serum plus 75 µl of anti-Pvs25 mouse blood serum was added (1:2).
4. 131 µl of healthy type AB blood serum plus 19 µl of anti-Pvs25 mouse blood serum was added (1:8).
5. 145.3 µl of healthy type AB blood serum plus 4.7 µl of anti-Pvs25 mouse blood serum was added (1:32).
6. 75 µl of healthy type AB blood serum plus 75 µl of anti-Pvs28 mouse blood serum was added (1:2).
7. 131 µl of healthy type AB blood serum plus 19 µl of anti-Pvs28 mouse blood serum was added (1:8).
8. 145.3 µl of healthy type AB blood serum plus 4.7 µl of anti-Pvs28 mouse blood serum was added (1:32).

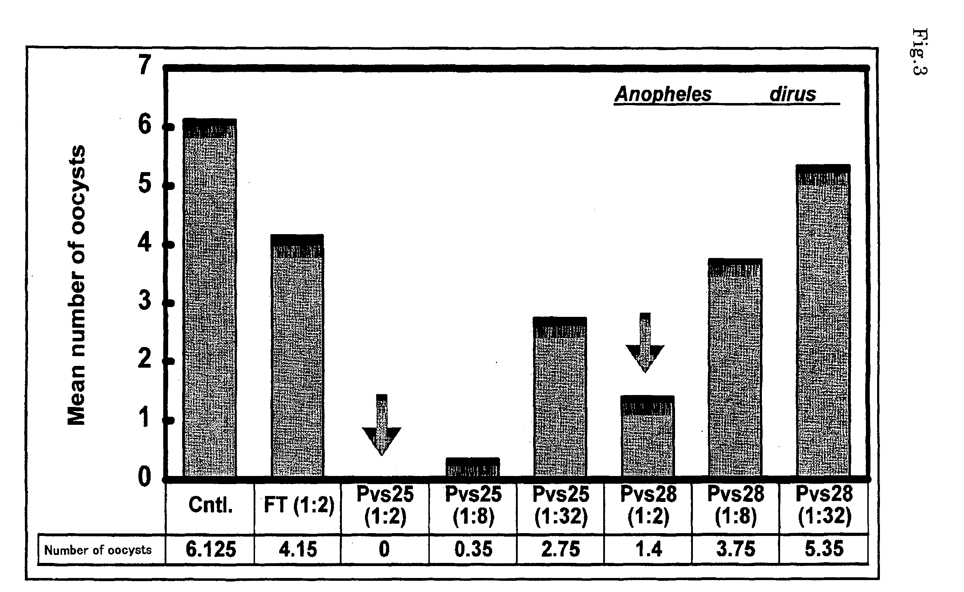

Mosquitos which sucked blood were collected to eliminate the other mosquitos which did not suck, taken back to the U.S. Forces medical research institute in Thailand (Bangkok), fed at 26 °C for one week, dissected microscopically after grouped into every twenty mosquitos, and subjected to counting in number of plasmodiums (oocysts) infecting on their midgut surfaces to give an indicator of the activity for preventing propagation. Fig. 3 is the sum of the data obtained from two patients (totally each group consists of 40 mosquitos).

The principle of a vaccine for preventing the propagation of malaria is as follows: a human is immunized with a protein (Pfs25, Pvs25 or Pvs28) expressed specifically on the surface of a plasmodium (mainly from zygote to ookinete) on the stage of living within a mosquito midgut to produce a specific antibody, which is then caused to take an antigen-antibody reaction with the surface protein in the midgut of the mosquito which sucked blood of the human, allowing inhibition of the protozoa from proliferation. As a result, the next growing stage, an oocyst is inhibited from emerging inside the mosquito, resulting in blocking the mediator mosquito from propagating malaria.
Therefore, to confirm that anti-serum produced from mice has the activity of a vaccine for preventing the propagation of malaria, experiments were conducted using Plasmodium vivax as a model. As a result, as seen in Fig. 3, a mosquito which sucked anti-FT mouse blood serum (1:2) had a mean oocyst number of 4.15, while no oocyst-infected mosquito was found in anti-Pvs25 mouse blood serum (1: 2) group, indicating that this antiserum had an extremely high activity for preventing propagation. This antiserum, even if diluted to 1:8, kept its activity to reduce an average oocyst number to 0.35 significantly (P<0.0001). Further, anti-Pvs28 mouse blood serum (1:2) also was found to have significant activity for preventing propagation (P<0.02), though the activity was weak compared with that of anti-Pvs25 mouse blood serum.

### EXAMPLE 5

### Confirming Activity of Anti-Pfs25-3D7 or Anti-Pfs25-TBV Mouse Blood Serum for Preventing Propagation of Malaria

The activity for preventing the propagation of malaria with anti-Pfs25-3D7 mouse blood serum was assayed to confirm by the same method as in Example 4. Further, anti-Pfs25-TBV mouse blood serum also was assayed. As described above, Pfs25 which had AT-rich codon was adjusted to prepare Pfs25-TBV, which allowed synthesize of Pfs25 in a yeast protein synthesis system. Furthermore, in place of anti-FT mouse blood serum (negative control) in Example 4, an adjuvant blood serum (a mouse blood serum obtained by immunizing a mouse with a mixture of PBS buffer and adjuvant) was used as a negative control.

1. 150 µl of healthy type AB blood serum was added.
2. 75 µl of healthy type AB blood serum plus 75 µl of Adjuvant blood serum (negative control) was added.
3. 75 µl of healthy type AB blood serum plus 75 µl of anti-Pfs25-3D7 mouse blood serum was added (1: 2).
4. 131 µl of healthy type AB blood serum plus 19 µl of anti-Pfs25-3D7 mouse blood serum was added (1: 8).
5. 145.3 µl of healthy type AB blood serum plus 4.7 µl of anti-Pfs25-3D7 mouse blood serum was added (1: 32).
6. 75 µl of healthy type AB blood serum plus 75 µl of anti-Pfs25-TBV mouse blood serum was added (1: 2).
7. 131 µl of healthy type AB blood serum plus 19 µl of anti-Pfs25-TBV mouse blood serum was added (1: 8).
8. 145.3 µl of healthy type AB blood serum plus 4.7 µl of anti-Pfs25-TBV mouse blood serum was added (1: 32).

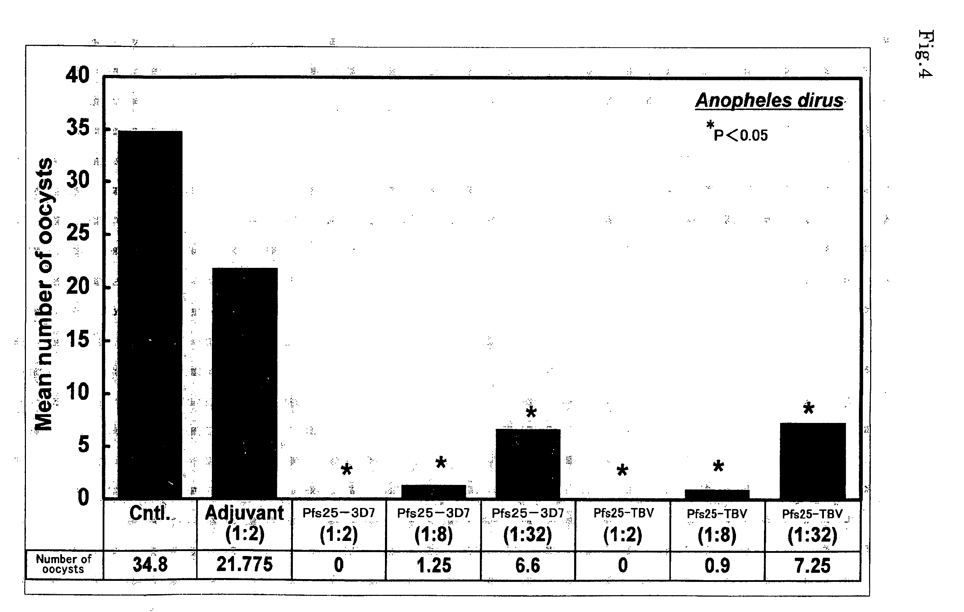

The results were shown in Figure 4. A mosquito which sucked adjuvant blood serum (1:2) had an average oocyst number of 21.775, while no oocyst-infected mosquito was found in anti-Pfs25-3D7 mouse blood serum (1:2) group, indicating that this antiserum had an extremely high activity for preventing propagation. This antiserum, even if diluted to 1:8, kept its activity to reduce an average oocyst number to 1.25 significantly (P<0.05). Further, anti-Pfs25-3D7 mouse blood serum was found to have significant activity for preventing propagation, which was equal compared with that of anti-Pfs25-TBV mouse blood serum.
Synthesis of Pfs25 in a yeast protein synthesis system needed alteration of the codons to adjust an AT content because Pfs25 gene is AT-rich. Further, a protein synthesis system derived from E. Coli often can not express an antigenic component with the retained antigenicity. It was confirmed that the method for producing an antigenic component using a cell-free protein synthesis means with the use of a wheat embryo extract according to the present invention could synthesize an antigenic component with the retained antigenicity independently of an AT content, which was different in performance from a conventional protein synthesis system.

The above results show that an antigenic component produced by the present invention can not only induce antibodies which recognize the original protein of protozoa, but also produce an effective antibody which has an activity for preventing propagation of the protozoa among those antibodies. Accordingly, it is revealed that a cell-free protein synthesis system with the use of a wheat embryo is useful for producing a vaccine antigenic component for preventing the propagation of malaria.

This application claims priority from Japanese Patent Application No. 2003-333659, which is herein incorporated by reference.

### Brief Description of the Drawings

Fig. 1 shows an electrophoresis representing the expression of malaria antigen using a cell extract solution for cell-free protein synthesis with the use of a wheat embryo of the present invention. From left, Pfs25-TBV, Pfs25-3D7, Pvs25, and Pvs28, wherein the result of each antigen is shown. In the figure, T: total, 1: non-adsorbed fraction, 2: purified protein, 3: washed fraction, and * shows an aimed size portion.
Fig. 2 shows the result of the immunostaining of ookinete of Plasmodium vivax to anti-Pvs28 blood serum.
Fig. 3 shows the activity of anti-Pvs25 or Pvs28 mouse blood serum for preventing vivax malaria propagation.
Fig. 4 shows the activity of anti-Pfs25-3D7 or anti-Pfs25-TBV mouse blood serum for preventing falciparum malaria propagation.

## Claims

1. A method for producing an antigenic component using a cell-free protein synthesis means with the use of a wheat embryo extract.

2. A method for producing an antigenic component which is encoded by a gene having an AT content of 50% or more by a base sequence of its protein-coding region, using a cell-free protein synthesis means with the use of a wheat embryo extract.

3. A method for producing an antigenic component derived from a plasmodium using a cell-free protein synthesis means with the use of a wheat embryo extract.

4. The method for producing according to any one of the claims 1 to 3, wherein the cell-free protein synthesis means uses a wheat embryo extract which is substantially freed from an endosperm and a low molecular synthesis inhibitor.

5. An antigenic component obtained by the method for producing according to the claim 4.

6. The antigenic component according to the claim 5, wherein the antigenic component has an amino acid sequence which has the identity or a homology of 70% or more to the amino acid sequence of SEQ. ID. No: 2, 4, 6 or 8.

7. The antigenic component according to the claim 6, wherein the antigenic component retains the antigenicity.

8. A method for manufacturing a vaccine using the antigenic component according to any one of the claims 5 to 7.

9. A vaccine obtained by the method for manufacturing according to the claim 8, wherein the vaccine comprises the antigenic component.

10. An antibody prepared by the vaccine according to the claim 9.

11. An antibody which reacts immunologically with the antigenic component according to any one of the claims 5 to 7.

12. A diagnostic agent comprising the antibody according to the claim 10 or 11.

13. A diagnostic kit comprising the diagnostic agent according to the claim 12.

14. A method for screening an antigenic component using a cell-free protein synthesis means with the use of a wheat embryo extract, comprising:
1) a step for preparing a gene comprising a region selected within a desired candidate gene,
2) a step for synthesizing a mRNA from the gene prepared in step 1),
3) a step for synthesizing a polypeptide by a cell-free protein synthesis means with the use of a wheat embryo extract using the mRNA synthesized in step 2) as a translation template, or synthesizing a polypeptide by a cell-free protein synthesis system with an integrated transcription/translation manner using the gene prepared in step 1) as a template,
4) a step for immunizing a mammal with the polypeptide synthesized in step 3) to obtain the antiserum,
5) a step for reacting the antiserum described above with a desired target microbe or a protein derived from the microbe to analyze the reactivity, and
6) a step for identifying the region selected within the candidate gene from which the antiserum has been derived as a candidate antigenic component if the antiserum is confirmed to react.
